# EUROPÄISCHE PATENTSCHRIFT

(11) **EP 2 219 684 B1**
(45) Veröffentlichungstag und Bekanntmachung des Hinweises auf die Patenterteilung: **16.07.2014**
(21) Anmeldenummer: 08854596.7
(22) Anmeldetag: 26.11.2008
(51) Int. Cl.: A61L 2/03, D06F 35/00, A47L 11/00

(54) **VORRICHTUNG ZUR REINIGUNG VON BODEN-OBERFLÄCHEN MIT ELEKTROLYSIERTEM WASSER MITTELS OXIDATIVER RADIKALE, ERZEUGT DURCH DIAMANT-ELEKTRODEN**
DEVICE FOR FOR CLEANING FLOOR SURFACES WITH ELECTROLYSED WATER BY MEANS OF OXIDATIVE RADICALS PRODUCED BY DIAMOND ELECTRODES
APPAREIL POUR PURIFIER DES SURFACES DE SOL AVEC DE L'EAU ÉLECTROLYSÉE PAR DES RADICAUX OXYDATIFS GÉNÉRÉS PAR DES ÉLECTRODES DE DIAMANT

(30) Priorität: 30.11.2007 CH 18532007
(43) Veröffentlichungstag der Anmeldung: 25.08.2010
(73) Patentinhaber: pro aqua BIOCLEAN GmbH & Co KG, 8712 Niklasdorf (AT)
(72) Erfinder: Steffen, Hanspeter, 3473 Alchenstorf (CH)
(74) Vertreter: Vinazzer, Edith
(86) Internationale Anmeldenummer: PCT/CH2008/000501
(87) Internationale Veröffentlichungsnummer: WO 2009/067838

(56) Entgegenhaltungen:
- EP-A- 0 994 074
- EP-A- 1 468 965
- EP-A- 1 944 403
- WO-A-2004/065682
- US-A- 4 434 629
- US-A1- 2003 141 202
- US-A1- 2005 186 345

## Beschreibung

### Technisches Gebiet

Die Erfindung betrifft eine Bodenreinigungsmaschine zw Chemie- und Rückstand-freien Reinigung, Hygienisierung, Desinfektion und Geruchsneutralisation von Bodenoberflächen mit elektrolysiertem kaltem oder warmem Wasser mittels Oxidativer Radikale erzeugt durch Bor gedopte Diamant-Elektroden.

### Stand der Technik

Bis anhin konnten Wäsche und Textilien nur mit Waschmitteln, chemischen Produkten und Desinfektionsmitteln oder mit Heisswasser hygienisch gereinigt werden, was sehr kostspielig ist und zudem eine enorme Belastung für die Umwelt durch Chemie enthaltende Abwässer darstellt, die schwierig zu reinigen sind, und biologische Reinigungsstufen in der Abwasserreinigung belasten.

Eine Lösung dieses Problems ist beispielsweise in der WO 2004/065682 A1 anhand einer Waschmaschine beschrieben welche mit einer alkalischen Elektrolyselösung und daher ohne herkömmliches Waschmittel funktioniert. Es wird einzig im Wasser gelöstes NaCl in einer Konzentration von 0.75 - 2g7l als Elektrolyt verwendet. Der verwendete Elektrolyse-Generator umfasst eine Membran zur Trennung der alkalischen und der sauren Elektrolyselösung. Die alkalische Lösung_wird zur Reinigung verwendet, während die saure Lösung entweder abgelassen oder gemischt mit der alkalischen Lösung als Spülwasser verwendet wird. Als Material für die Anode wird rostfreier Stahl, Weichstahl, Ni, Ti, Ir, Ru oder Oxide davon verwendet.

Eine Waschmaschine, welche ebenfalls Elektrolyse anwendet, wird in der US4,434,629 beschrieben, Hier wird jedoch nicht mit der Elektrolyselösung, gereinigt, sondern es wird in einer Elektrolysezelle Chlorgas aus einer NaCl-Lösung produziert. Dieses Chlorgas wird als Bleichmittel im Waschwasser gelöst. Das Waschwasser wird dadurch leicht sauer, was ein besseres Bleichergebnis bewirkt. Bei der verwendeten Elektrolysezelle handelt es sich ebenfalls um eine zweikammrige Zelle.

Ein Verfahren zur Wasserdesinfektion durch Elektrolyse wird in der US2003/0141202 A1 beschrieben. Es wird die basische Elektrolyselösung verwendet. Bei der verwendeten Anode handelt es sich um eine Elektrode mit einer leitenden Dismantbeschichtung. Das Verfahren kann auch zur Desinfektion von Spülwasser für Kleiner verwendet werden,

Ein ähnliches Verfahren zur anodischen Oxidation von wässrigen Lösungen wird in der EP 0994 074 A2 beschrieben. In diesem Verfahren wird ebenfalls eine mit Diamant beschichtete Elektrode verwendet, an welche eine Spannung angelegt wird. Diese Spannung liegt unterhalb des Potentialbereichs, bei welchem eine gössere Sauerstoffentwicklung an der Elektrode erfolgt. Die Diamantbeschichtung der Elektrode ist mit Bor dotiert und dadurch leitend gemacht. Solche Bor dotierte Elektroden sind auch aus der US 2005/0186345 A1 sowie der EP 1 468 965 A1 bekannt. Nebst Bor kann laut diesen Dokumenten auch Phosphor verwendet werden.

Mit der neuen Erfindung soll aufgezeigt werden, dass mit Hilfe von elektrolysiertem Wasser mittels Oxidativen Radikalen, die mit Diamant Elektroden, Dank deren speziellen elektrochemischen Eigenschaften, erzeugt werden, mittels einer ultraschnellen Superoxidation Wäsche und Textilien gereinigt, hygienisiert, und desinfiziert werden können, ohne den teuren Einsatz von Umwelt belastenden Waschmitteln und toxischen Chemikalien und Energie verschwendendem Heisswasser. Die zu diesem Zweck benötigte Reinigungs-Intensität mittels Oxidativer Radikale kann nur mit Hilfe von Diamant Elektroden mit 4 Volt Überspannung erreicht werden.

### Darstellung der Erfindung

Aufgabe der Erfindung ist die Angabe einer kostengünstigen, effizienten, Umwelt freundlichen, biologischen und Rückstand freien Bodenreinigungsmaschine unter Verwendung von elektrolysiertem Wasser mittels Oxidativen Radikalen, die mit Diamantelektroden erzeugt werden, Dank einer ultraschnellen Superoxidation hygienisch zu reinigen und zu desinfizieren. Die Erfindung wird durch Anspruch 1 definiert.

### EINFÜHRUNG

### Elektrolytisch hergestelltes, oxidatives Wasser (EOW)

Elektrolytisch oxidatives Wasser (EOW) oder chemisch aktives Wasser reinigt durch Ionen-Aktivität und zerstört Mikroorganismen, wie Viren, Bakterien, Pilze, Hefen und Einzeller durch oxidative Radikale nicht chemisch, sondern physikalisch.

Wegen seines hohen oxidativen Reduktionspotentials (ORP) beschädigt "AktivesWasser" die Zellwand-Membranen von Pathogenen.

Der Krankheitserreger ist komprimitiert, was zu einer osmotischen oder hydrogenen Überlastung im Zellinneren führt.

Die beschädigten Zellmembranen erlauben einen erhöhten Wassertransfer zwischen den Zellmembranen, was zu einer hydrogenen Überflutung der Zellen führt, und diese schneller gefüllt werden, als die Zellen sich des Wassers entledigen können.

Diese Tatsache führt zu einem Zerplatzen der Zellen, respektive zum Zelltod durch Druckexplosion in wenigen Sekunden.

Da es sich um ein physikalisches Zerstörungsprinzip handelt,
ergeben sich nachweislich keine Resistenzen bei Pathogenen.

### Prinzip der Elektrolyse (verg. Fig. 1)

### Beispiel einer Elektrolyse mit einer Zinkiodid - Lösung (Elektrodenmaterial beliebig)

Verbindet man zwei Metallplättchen (Elektroden) mit jeweils einem Kabel und einer Vorrichtung die Gleichstrom erzeugt z.B. einer Batterie oder einem Gleichrichter - und überführt diese Plättchen in ein Becherglas mit wässriger Lösung (Beliebige Ionen) und legt nun eine Spannung an, so bildet sich an beiden Metallplättchen ein Stoff, dessen Ionen in der Lösung vorhanden sind.

Die Spannungsquelle bewirkt einen Elektronenmangel in der mit dem Pluspol (Anode) verbundenen Elektrode und einen Elektronenüberschuss in der anderen, mit dem Minuspol (Kathode) verbundenen Elektrode. Die wässrige Lösung zwischen der Kathode und Anode enthält Elektrolyte, das sind positiv oder negativ geladene Ionen. Die positiv geladenen Kationen in einer Elektrolysezelle wandern durch das Anlegen einer Spannung zur negativ geladenen Kathode (Anziehung entgegen gesetzter Ladungen). An der Kathode nehmen sie ein oder mehrere Elektronen auf und werden dadurch reduziert.

An der Anode läuft der entgegen gesetzte Prozess ab. Dort geben die negativ geladenen Anionen Elektronen ab, das heißt sie werden oxidiert. Die Zahl der durch die Reduktion an der Kathode verbrauchten Elektronen entspricht den von der Anode aufgenommenen Elektronen. Bei der Elektrolyse von wässriger Kochsalzlösung entsteht die gleiche Volumenmenge Wasserstoffgas wie Chlorgas. Bei der Elektrolyse von Wasser entsteht doppelt so viel Wasserstoffgas wie Sauerstoffgas, da die zwei positiv geladenen Protonen eines Wassermoleküls zur Kathode wandern und dort jeweils ein Elektron aufnehmen müssen damit sich Wasserstoff bildet, während das doppelt negativ geladene Sauerstoffanion an der Anode gleich zwei Elektronen abgeben muss, um sich zum Sauerstoffmolekül zu verbinden.

Die Spannung, die zur Elektrolyse mindestens angelegt werden muss, bezeichnet man als Abscheidungspotential, bei der Elektrolyse von Wasser oder bei wässrigen Salzlösungen spricht man auch von der Zersetzungsspannung. Diese Spannung (oder eine höhere Spannung) muss angelegt werden, damit die Elektrolyse überhaupt abläuft. Für jeden Stoff, für jede Umwandlung von Ionen zu zwei oder mehratmigen Molekülen kann die Zersetzungsspannung, das Abscheidpotential anhand des Redoxpotentials ermittelt werden. Aus dem Redoxpotential erhält man noch viele andere wichtige Hinweise für die Elektrolyse, beispielsweise zur elektrolytischen Zersetzung von Metallelektroden in Säure oder zur Verminderung von Zersetzungsspannung durch Abänderung von pH-Werten.

Beispielsweise lässt sich durch das Redoxpotential berechnen, dass die Bildung von Sauerstoff an der Anode bei der Elektrolyse von Wasser in basischer Lösung (Zersetzungsspannung: 0,401 V) unter geringerer Spannung abläuft als in saurer(Zersetzungsspannung: 1,23 V) oder neutraler (Zersetzungsspannung: 0,815 V) Lösung, an der Kathode hingegen bildet sich leichter Wasserstoff unter sauren Bedingungen, als unter neutralen oder basischen Bedingungen).

Sind in einer Elektrolytlösung mehrere reduzierbare Kationen vorhanden, so werden nach der Redoxreihe zunächst die Kationen an der Kathode reduziert, die in der Redoxreihe (Spannungsreihe) ein positiveres (schwächer negatives)Potential haben, die also dem 0 Potential der Proton-Wasserstoff Elektrodenspannung möglichst nahe kommen. Bei der Elektrolyse einer wässrigen Kochsalzlösung bildet sich an der Kathode normalerweise Wasserstoff und nicht Natrium. Auch beim Vorliegen von mehreren Anionenarten, die oxidiert werden können, kommen zunächst diejenigen zum Zuge, die in der Redoxreihe möglichst nahe am Spannungsnullpunkt, also ein schwächeres positives Redoxpotential besitzen. Normalerweise entsteht bei der Elektrolyse von wässriger NaCl an der Anode also Sauerstoff und nicht Chlor. Nach Überschreiten der Zersetzungsspannung wächst mit Spannungszunahme proportional auch die Stromstärke. Nach Faraday ist die Gewichtsmenge eines elektrolytisch gebildeten Stoffs proportional zu der geflossenen Strommenge (Stromstärke multipliziert mit der Zeit). Für die Bildung von 1 g Wasserstoff (ca. 11,2 Liter, bei der Bildung eines Wasserstoffmoleküls werden zwei Elektronen benötigt) aus wässriger Lösung wird eine Strommenge von 96485 C (As)=1Faraday benötigt. Bei einer Stromstärke von 1 A zwischen den Elektroden dauert die Bildung von 11,2 Litern Wasserstoff also 26 Stunden und 48 Minuten.

Neben dem Redoxpotential ist noch die Überspannung (das Überpotential) von Bedeutung. Auf Grund von kinetischen Hemmungen an Elektroden benötigt man häufig eine deutlich höhere Spannung als sich dies aus der Berechnung der Redoxpotentiale errechnet. Die Überspannungseffekte können je nach Materialbeschaffenheit der Elektroden - auch die Redoxreihe ändern, so dass andere Ionen oxidiert oder reduziert werden als dies nach dem Redoxpotential zu erwarten gewesen wäre. Kurz nach Abschaltung einer Elektrolyse kann man mit einem Amperemeter einen Stromausschlag in die andere Richtung feststellen. In dieser kurzen Phase setzt der umgekehrte Prozess der Elektrolyse, die Bildung einer galvanischen Zelle ein. Hierbei wird nicht Strom für die Umsetzung verbraucht, sondern es wird kurzzeitig Strom erzeugt; dieses Prinzip wird bei Brennstoffzellen genutzt.

Wenn man durch eine Elektrolyse eine Trennung einzelner Moleküle oder Bindungen erzwingt, wirkt gleichzeitig ein galvanisches Element, dessen Spannung der Elektrolyse entgegenwirkt. Diese Spannung wird auch als Polarisationsspannung bezeichnet.

### Elektroden

Es gibt nur wenige Anoden-Elektroden, die während der Elektrolyse innert bleiben - also überhaupt nicht in Lösung gehen. Platin, Kohle resp. Diamant sind Materialien, die sich während einer Elektrolyse überhaupt nicht auflösen. Es gibt auch Metalle, die sich trotz stark negativem Redoxpotentials nicht auflösen. Dies wird als "Passivität" bezeichnet. Eine Eisenanode, die mit konzentrierter Salpetersäure behandelt wurde, löst sich nicht auf und es gehen keine Eisen (II) oder (III)-Kationen in Lösung; sie hat "Passivität".

Hemmungserscheinungen an der Anode, die bei der Sauerstoffbildung zu einer Überspannung führen, beobachtet man vor allem bei Diamantanoden (Überspannung: 0,44 V). Bei diesen entsteht bei der Elektrolyse von wässriger Kochsalzlösung Chlor statt Sauerstoff. An Zink-, Blei-(Überspannung: 0,78 V) und besonders Quecksilberkathoden (0,80 V) zeigen Wasserstoffprotonen eine erhebliche Überspannung und die Bildung von Wasserstoff erfolgt erst bei einer viel höheren Spannung. Die erhebliche Überspannung von Wasserstoff an der Quecksilberkathode, in der das Natrium als Amalgam gebunden wird und daher dem Gleichgewicht entzogen wird, nutzt man zur technischen Herstellung von Natronlauge. Durch die erhebliche Überspannung an dieser Elektrode bei der Wasserstoffbildung ändert sich die Redoxreihe und statt Wasserstoffprotonen wandern nun Natriumkationen zur Quecksilberkathode.

### Elektrolyse von Wasser

Die Elektrolyse von Wasser besteht aus zwei Teilreaktionen, die an den beiden Elektroden ablaufen. Die Elektroden tauchen in Wasser ein, welches durch die Zugabe von etwas Kochsalz besser leitend gemacht wird, wobei dann anstatt Sauerstoff Chlor gewonnen wird.

Positiv geladene Hydronium-Ionen (H30+) wandern im elektrischen Feld zu der negativ geladenen Elektrode (Kathode), wo sie jeweils ein Elektron aufnehmen. Dabei entstehen Wasserstoff-Atome, die sich mit einem weiteren, durch Reduktion entstandenen H-Atom zu einem Wasserstoff-Molekül vereinigen. Übrig bleiben Wasser-Moleküle.

2 H₃O⁺ + 2 e⁻ → H₂ + 2 H₂O

Der abgeschiedene, gasförmige steigt an der Kathode auf.

Zur positiv geladenen Elektrode (Anode) wandern die negativgeladenen Hydroxid-Ionen.

Jedes Hydroxid-Ion gibt ein Elektron an den Plus-Pol ab, so dass Sauerstoff-Atome entstehen, die sich zu Sauerstoff-Molekülen vereinigen resp. bei NaCl Zugabe zu Chlor-Molekülen.

Die übrig bleibenden H⁺-Ionen werden umgehend von Hydroxid-Ionen zu Wasser-Molekülen neutralisiert.

4 OH⁻ → O₂ + 2 H₂O + 4 e⁻

Auch hier steigt der abgeschiedene Sauerstoff als farbloses Gas an der Anode auf. Die Gesamtreaktionsgleichung der Elektrolyse von Wasser lautet:

4 H₃O⁺ + 4 OH⁻ → 2 H₂ + O₂ + 6 H₂O

Die auf der linken Seite stehenden Hydronium- und Hydroxid-Ionen entstammen der Autoprotolyse des Wassers:

8 H₂O → 4 H₃O⁺ + 4 OH⁻

Man kann die Elektrolysegleichung daher auch folgendermaßen schreiben:

8 H₂O → 2 H₂ + O₂ + 6 H₂O

bzw. nach Kürzen des Wassers:

2 H₂O → 2 H₂ + O₂

### Hydroxidion

Das Hydroxidion ist ein negativ geladenes Ion, das entsteht, wenn Basen mit Wasser reagieren. Seine chemische Formel lautet OH⁻.

Eine allgemeine Base B reagiert nach folgendem Schema mit Wasser:

B + H₂O ⇄ HB⁺ + OH⁻

Anhand der Konzentration der Hydroxidionen kann man den pH-Wert der entstandenen Lösung ermitteln. Dazu berechnet man erst den so genannten pOH-Wert.

*pOH* = - log *c*(OH⁻)

Und daraus den pH-Wert:

*pH= k- pOH*

*Zu jeder Temperaturgibt es jeweils ein k.*

*Unter Normbedingungen ist k= - 14.*

Hydroxidionen sind auch in reinem Wasser bei 20°C in einer Konzentration von 10⁻⁷ mol · l⁻¹ enthalten. Das hängt mit der Autoprotolyse des Wassers nach folgender Reaktionsgleichung zusammen:

H₂O + H₂O ⇄ H₃O⁺ + OH⁻

Dank dem Ueberpotential Fenster von 4 Volt ist eine Bor gedopte Diamantelektrode fähig, auch Mineralien im Wasser zu oxidieren. Dabei entstehen Calcium-Peroxyd, Schwefelperoxyd, Phosphorperoxyd etc., die ebenfalls extrem starke Oxydationsmittel sind und die durch keine anderen Elektroden-Typ erreicht werden kann, siehe Fig. 2.

### Zulassung

Frühe eigene Versuche und Versuchsergebnisse führten zur Einreichung von Bewilligungs-Gesuchen bei der FDA (Food and Drug Administration, USA), welche im Dezember2002 die Bewilligung für die neue Technologie erteilte und mit dem Status"GRAS" (Generally Regarded as Safe) auszeichnete.

Elektrolysiertes oxidatives Wasser erhielt FDA (USA Food and Drug Administration), USDA (United Status Department of Agriculture) und EPA (USA Environmental Protection Agency) - Zulassung für allgemeine Applikationen im Nahrungsmittel-Bereich, für die Nahrungsmittel-Oberflächen Desinfektion, für Milch-, Fleisch- und Restaurant- technische Anwendungen.

Die entsprechenden Seiten der Bewilligungsnummern der FDA und USDA lauten 21 CFR 173, 178, 182, 184 & 198.

Die EPA Bewilligungs- und Publikations-Seite lautet 40 CFR 180.940 und die des National Organic Programms ist 21 CFR 178.1010.

### Darstellung der Verfahrenskomponenten

Das Verfahren beinhaltet folgende Technischen Hilfsmittel und Prozessschritte:

### Technische Hilfsmittel

1. Zur Durchführung des Verfahrens sind nötig:
   1 WASCHMASCHINE für TEXTILIEN standard
   2 GESCHIRRSPUEHLER standard
   3 BODENREINIGUNGSMASCHINE standard
   4 WASCHANLAGE für FAHRZEUGE und/oder TIERE und oder andere Waschmaschinen
2. Elektrolyse Generator mit einer oder mehreren Elektrolysezellen, mit Vorzugsweise Bor beschichteten Diamant - Elektroden, einkammrig oder zweikammrig mit Diaphragma , Pumpe aus vorzugsweise korrosionsfreiem Stahl oder Kunststoff, Filter, Flussmeter, Druckregulierung mit vorzugsweise 2 Hähnen und 2 Manometern, elektrischer Wasser- Flusssensor, Elektronische Steuereinheit mit Zeit gesteuerter automatischer Elektroden-Umkehrpolarisation, Redox-Meter, Wasservorratstank mit Eintritts und Austrittshahn, Wasser Leitungen, Einweg-Rücklauf-Ventil. Elektronische programmierbare Steuereinheit mit Schalter, elektronische Wasser-Pegel-Kontrolle mit elektronischem Zufluss-Ventil, Ein- und Ausschalthebel. Zeitmess- und Schalt-Uhr, Wasser Zu- und Ableitungen zur Verbraucher-Einheit.
3. 1 WASSERTANK als RESERVOIR

### Herstellung der bioziden und reinigenden Oxidativen Radikale in wässeriger, salzhaltiger Lösung mittels Elektrolyse.

Die Herstellung der bioziden und reinigenden Oxidativen Radikale in wässriger, salzhaltiger Lösung kann durch 2 verschiedene Elektrolyseverfahren erfolgen.

Das erste Verfahren wird implementiert mit der Diamant Elektrolyse mittels Diamantbeschichteten Elektroden. Dabei entsteht ein Cocktail aus Oxidativen Radikalen nahe am "Neutralen Bereich" mit einem pH - Wert von 6,4 bis 6,8. An der Anode werden neben OH-Hydroxylgruppen und 03 vor allem freies Chlor (Cl-) gebildet, die mit den Hydroxylgruppen zur Bildung von Hypochlorid HOCL und Hypochlorid Säure H20CL führen, die organisch sehr rasch abgebaut werden. Um die Elektrolyse des Wassers bezüglich Stromverbrauchs günstiger und besser durchführen zu können, werden dem Wasser wegen der verbesserten Elektrokonduktivität NaCl Salz zugemischt.

Bei der Elektrolyse dieser Salzverbindungen entstehen zudem oxidierende

Moleküle, wie reduzierendes Peroxyd-Di-Sulfat, Peroxyd-Di-Phosphat und Percarbonat.

Die NaCl Salzkonzentration beträgt pro Liter Wasser:

Vorzugsweise 0,5-8 Gramm NaCl (Kochsalz) oder mehr.

Das zweite Verfahren wird implementiert mit der Zylinder Elektrolyse mit Diaphragma, wo die Elektrolyse Zellen voneinander getrennt sind, bestehend aus einer Anoden - Kammer und einer Kathoden - Kammer. An der positiven Anode aus vorzugsweise Bor gedoptem Diamant bilden sich Säure bildende negativ aufgeladene Anionen in einem sauren Bereich von ca. 2.4 pH mit negativer Ladung, und an der negativen Kathode bilden sich Basen bildende positive Kationen in einem alkalischen Bereich von ca. 11 pH mit einer positiven Ladung.

Diese zwei sauren und alkalischen wässrigen Elektrolysen Lösungen können jetzt beliebig gemischt werden und je nach Einsatz im sauren oder basischen Bereich eingesetzt werden.

Bei der Elektrolyse von reinem Wasser ohne Salz, werden folgende Oxidativen Radikale gebildet:

### ELECTROLYTISCHER PROZESS von Wasser

Es entstehen die verschiedensten oxidativen Radikale, wenn Wasser (H2O) elektrolysiert wird zum Beispiel: (E0 ist das Standart Redox-Potential)*:

O2 + H + e- HO2 E0 = - 0, 1 3 V [1]

2H+ + 2e- H2 E0 = 0.00 V [2]

H02 + H+ + e- H2O2 E0 = +1.50 V [3]

03 + 2H+ + 2e- 02 + H2O E0 = +2.07 V [4]

OH- + H+ + e- H2O E0 = +2.85 V [5]

H2O + e- H+ OH- E0 = - 2.93 V [6]

OH+ e- OH- E0 = +2.02 V [7]

### ELECTROLYTISCHER PROZESS von Wasser mit Salz NaCl

An der Kathoden - Seite

Na+ + e- Na

2Na + 2H2O 2Na+ + 20H- + H2

An der Anoden - Seite

2Cl- - 2e- Cl2

Es muss hier erwähnt werden, dass Cl2 (Chlorgas) and OH- wie folgt reagieren:

Cl 2 + 20H- Cl O- + Cl- + H2O

oder

Cl 2 + OH- HCIO + Cl-

### LOESUNG DER AUFGABE

Die Lösung der Aufgabe ist durch die Merkmale der unabhängigen Patent - Ansprüche definiert.

Gemäss der Erfindung zeigt die Bodenreinigungsmaschine zur Chemie- und Rückstand freien Reinigung, Hygienisierung, Desinfektion und Geruchsneutralisation von Bodenoberflächen mittels elektrostatischer Spraytechnolgie und unter Verwendung von elektrolysiertem kaltem oder warmem Wasser und mit Hilfe von Oxidativen Radikalen, produziert mit Diamant-Elektroden, die Art und Weise der Biozide, insbesondere der spezifischen Eigenschaften des elektrolysierten, Oxidativen Wassers, dessen Herstellung, dessen Salzkonzentration und Salzzusammensetzung, dessen Redoxpotential, respektive dessen Konzentration in freien oxidativen Radikalen und Gesamtkonzentration der Oxidativen Radikale, und dessen pH-Wert und Aufwandmenge für einen effizienten Reinigungsvorgang auf.

Die Erfindung bildet ein integriertes System, in welchem die technischen Komponenten der Oxidativen Radikalen-Herstellung- mittels Diamant- Elektroden im Wasser und Zwischenlagerung im Vorratstank, mit den entsprechenden Applikations-Geraeten auch in Kombination mit elektrostatischer Spraytechnologie zur - Reinigung und Desinfektion und Geruchsneutralisation von Bodenoberfiächen mit elektrolysiertem, oxidative Radikale enthaltendem, kaltem oder warmem Wasser integriert sind.

Dabei liegt der Schwerpunkt der Innovation nicht nur in der technischen Kombination der mit einer Elektrolysen Anlage mit Bor gedopten Diamant Elektroden zur Herstellung von Oxidativen Radikalen, sondern auch in der neuen Verfahrens- und Applikations- Technik der kombinierten Anwendung von elektrolytischen Wasch - Anwendungsanlagen mit einer wässrigen Lösung aus Oxidativen Radikalen, die Dank dem speziellen Überspannungspotential der Bor gedopten Diamant-Elektroden und mittels einer ultraschnellen Superoxidation, nicht nur reinigen sondern auch desinfizieren können und sogar in der Lage sind Biofilme aufzulösen und ohne Chemie, ohne toxische Substanzen, rückstandsfrei arbeiten zur Entlastung der Umwelt.

In mehrjährigen Versuchen wurden die optimalen Konzentrationen von Oxidativen Radikalen im Wasser und die spezifischen elektrolytischen Parameter wie Ladungsdichte, Spray- und Waschflüssigkeits- Aufwand und Druckanforderungen und Behandlungszeiten eruiert, um eine perfekte Reinigung und Desinfektion auf allen Arten von Oberflächen, Materialien und Oberflächen, zu erreichen.

Der Erfinder hat in mehrjähriger Forschungs- und Entwicklungsarbeit im Labor und im praktischen Einsatz das neue Verfahren getestet und perfektioniert und eine Effizienz von nahe 100% erreicht.

Nach Kenntnisstand des Erfinders sind bis heute keine wissenschaftlichen Arbeiten auf dem Gebiet der Desinfektion und Reinigung von Wäsche, Textilien, Geschirr, Bodenoberflächen und Fahrzeugen anderen Oberflächen, Materialien und Gegenständen mittels der Kombination von mit Bor gedopten Diamant Elektroden, Dank ausserordentlichen elektrochemischen Eigenschaften, elektrolytisch aus Wasser erzeugten oxidativen Radikalen als Biozide und Reinigungsmittel gegen Schmutz, Keime, Schimmelpilze, Viren und Bakterien etc. , und verschiedenen Applikations-Waschmaschinen und mittels elektrostatischer Applikations-Technologie, als in die Tiefe wirkende Reinigungs- und Desinfektions-Technik bekannt, noch wird eine gleichartige Technologie unter Verwendung von Diamant Elektroden für den selben Zweck heute irgendwo eingesetzt.

### Ausführung der Erfindung

Die Erfindung soll an einem Beispiel einer Bodenreinigungsmaschine bestehend aus einem Elektrolysen-Generator mit Diamant Elektroden, einem Zwischentank und einer Bodenreinigungsmaschine.

### Textil Waschmaschine, Geschirrspüler, Bodenreinigungsmaschine, Waschanlage für Fahrzeuge und Tiere.

Eine konventionelle Waschmaschine, oder Geschirr-Spüler, Bodenreinigungsmaschine, Waschanlage für Fahrzeuge betrieben mit Wasser, das elektrolytisch gewonnene Oxidative Radikale enthält zur Reinigung und Hygienisierung von Textilien aller Art setzt sich aus folgenden technischen Einzelteilen zusammen:
1. Handelsübliche Textil-Waschmaschine, 240 V 50 Hz, Geschirrspüler, Bodenreinigungsmaschine, Waschmaschine für Fahrzeuge und oder Tiere.
2. Elektrolyse Generator mit vorzugsweise einer oder mehreren ein-kammrigen Electrolysezellen, parallel geschaltet, mit Bor gedopten Diamant-Elektroden , Pumpe aus korrosionsfreiem Stahl mit einer Schöpfleistung von vorzugsweise 600 Litern pro Stunde und vorzugsweise 4 bar Druck, Filter mit 50 mesh, Flussmeter bis 900 Liter pro Stunde, Druckregulierung mit vorzugsweise 2 Hähnen und 2 Manometern, elektrischer Wasser- Flusssensor, Elektronische Steuereinheit mit Zeit gesteuerter automatischer Elektroden-Umkehrpolarisation, Redox-Meter, Wasservorratstank mit Eintritts und Austrittshahn, Wasser- Leitungen, Einweg-Rücklauf-Ventil. Elektronische programmierbare Steuereinheit mit Schalter, elektronische Wasser-Pegel-Kontrolle mit elektronischem Zufluss-Ventil, Ein- und Ausschaltknopf. Zeitmess- und Schalt-Uhr, Wasser Zu- und Ableitungen inklusive Leitungsverbindung zum Zwischentank.
3. Zwischentank von vorzugsweise 500 oder mehr Litern Inhalt für Wasser mit Deckel, Entleerungsventil und Eingangs- und Ausgangsleitungen mit Hahnen und Verbindungsschlauch zu Sprühlanze oder. Sprüh-Düse/n

Als erster Arbeitsschritt wird das Gerät an das Stromnetz angeschlossen
220/240 V.

Das Elektrolysen-Gerät wird alsdann eingeschaltet.

Der Zwischentank mit 500 Liter Inhalt wird mit normalem Härte armem Wasser gefüllt und je nach Bedarf mit vorzugsweise 0.5 bis 8 Gramm Kochsalz pro Liter versetzt, d.h. mit bis zu 400 Gramm Kochsalz (NaCl).

Die vorprogrammierte Elektrolyse-Einheit wird jetzt eingeschaltet. Die Korrosion beständige Pumpe (600 Liter pro Stunde) pumpt nun das Wasser mit 10 Litern pro Minute durch die mit Diamant-Elektroden bestückten Elektrolysen-Zellen. Dort wird das Wasser über die Diamanten Elektroden (Anode/Katode) elektrolysiert und es entstehen Oxidative Radikale, die auf Oberflächen eine ultraschnelle Superoxidation bewirken, die reinigt und zu einer vollständigen Desinfektion und Abtötung von Mikroorganismen führt.

Das Wasser wird solange elektrolysiert, bis die gewünschte Konzentration hergestellt ist. Das programmierte REDOX Monitor-Gerät schaltet automatisch ein und aus, oder eine Schaltuhr steuert das Elektrolyse-Gerät.

Wenn die gewünschte Konzentration von Oxidativen Radikalen erreicht ist, bestimmt durch den gewünschten ORP-Wert (oxidatives Reduktions-Potential), kann das entsprechende Reinigungsgerät oder die Waschmaschine eingeschaltet werden.

Der Waschprozess läuft konventionell ab, aber mit Wasser, das reinigende oxidative Radikale enthält und keine Waschpulver oder Reinigungsmittel.

Das reduzierende Wasser wirkt wie ein Seifenprodukt und entfernt nicht nur Schmutz und bakterielle Schmutz- Bio-Filme, sondern desinfiziert ebenfalls durch die Abtötung von 99,9 % aller Mikroorganismen, wie Viren, Gramm positive und Gramm negative Bakterien, Hefen, Protozoen etc. in Sekunden.

Das oxidative Wasser hat eine verlängerte Wirkungszeit, was die Desinfektions- Intensität begünstigt. Die Reinigung ist perfekt und es entstehen keine toxischen Rückstände. Das Verfahren kann somit auch in CIP Anwendungen (Clean in Place) angewendet werden.

Das Reinigungs- Verfahren mit elektrostatischer Spraytechnologie und Oxidativen Radikalen ist billiger als jedes andere Reinigungs-Verfahren mit Chemie. Der Energieverbrauch beträgt lediglich 600 W/h für die Herstellung von 600 Litern Desinfektions- Lösung.

## Patentansprüche

1. Bodenreinigungsmaschine zur chemie- und rückstandsfreien Reinigung, Hygenisierung, Desinfektion und Geruchsneutralisation von Bodenoherflächen,
**dadurch gekennzeichnet,**
**dass** sie einen Elektrolysegenerator mit zumindest einer Elektrolysezelle mit Diamantelektroden, die mit Bor dotiert sind, aufweist, wobei der Elektrolyse Generator aus Wasser elektrolysiertes, kaltes oder warmes Wasser mit oxidativen Radikalen erzeugt.

2. Bodenreinigungsmaschine nach Anspruch 1, **dadurch gekennzeichnet, dass** die Elektrolysezelle einkammrig oder zweikammrig mit Diaphragma ausgeführt ist.

3. Bodenreinigungsmaschine nach einem der Ansprüche 1 oder 2, **dadurch gekennzeichnet, dass** sie eine Pumpe, einen Filter, ein Flussmeter, eine Druckregulierung, einen elektrischen Wasser-Flusssensor, eine elektronische Steuereinheit mit Zeit gesteuerter automatischer Elektroden-Umkehrpolarisation, ein Redox-Meter, einen Wasservorratstank mit Eintritts- und Austrittshahn, Wasserleitungen, ein EinwegRücklauf-Ventil, eine elektronische programmierbare Steuereinheit mit Schalter, eine elektronische Wasserpegelkontrolle mit elektronischem Zufluss-Ventil, Ein- und Ausschalthebel, eine Zeitmess- und Schalt-Uhr, Wasser Zu- und Ableitungen zu Hochdruckreiniger, ein Erdungskabel und Stromleitungen mit Stecker, einen Operationsstundenmesser, einen Notschalter und einen Sicherungskasten aufweist.

4. Bodenreinigungsmaschine nach einem der Ansprüche 1 bis 2, **dadurch gekennzeichnet, dass** sie ein Montagechassis, welches vorzugsweise fahrbar ist, aufweist.

5. Bodenreinigungsmaschine nach einem der Ansprüche 1 bis 2, **dadurch gekennzeichnet, dass** sie einen Wassertank mit Umwälzpumpe zur Zwischenlagerung aufweist.

6. Bodenreinigungsmaschine nach Anspruch 1, **dadurch gekennzeichnet, dass** sie eine elektrostatische Sprühanlage zum Applizieren des elektrolysierten Wassers aufweist.

7. Bodenreinigungsmaschine nach Anspruch 6, **dadurch gekennzeichnet, dass** die elektrostatische Sprühanlage aus korrosionsfreien Teilen besteht und elektronische Steuer- und Kontrolleinheiten aufweist und über eine Sprühlanze oder einen Sprühbalken mit verstellbaren, elektrostatischen, Luft assistierten Sprühdüsen verfügt.

8. Bodenreinigungsmaschine nach einem der Ansprüche 1 bis 6, **dadurch gekennzeichnet, dass** die Diamant-Elektroden geeignet sind ein Spannungsüberpotential von mindestens 4 Volt aufzuweisen.

9. Bodenreinigungsmaschine nach einem der Ansprüche 1 bis 8, **dadurch gekennzeichnet, dass** das Wasser im Vorratstank mit 0,5 g bis 8 g Kochsalz pro Liter Wasser versetzt ist.

## Claims

1. A floor cleaning machine for non-chemical and residue-free cleaning, sanitation, disinfection and odor neutralization of floor surfaces,
**characterized in**
**that** it comprises an electrolysis generator having at least one electrolysis cell with diamond electrodes that are doped with boron, wherein the electrolysis generator produces from water electrolyzed cold or warm water with oxidative radicals.

2. The floor cleaning machine according to claim 1, **characterized in that** the electrolysis cell is constructed as a single chamber type or a dual chamber type with a diaphragm.

3. The floor cleaning machine according to claim 1 or claim 2, **characterized in that** it comprises a pump, a filter, a flow meter, pressure regulation, an electrical water flow sensor, an electronic control unit with time-controlled automatic electrode polarity reversal, a redox meter, a water storage tank with an inlet cock and an outlet cock, water pipes, a one-way return valve, an electronic programmable control unit with a switch, an electronic water level control with electronic inflow valve, a switch-on and -off lever, a timer, water feed and discharge lines to the high-pressure cleaner, a grounding cable and power supply lines with plug, an operating-hour meter, an emergency switch and a fuse box.

4. The floor cleaning machine according to any one of the claims 1 to 2, **characterized in that** it has a mounting chassis that is preferably movable.

5. The floor cleaning device according to any one of the claims 1 to 2, **characterized in that** it comprises a water tank with a circulation pump for temporary storage.

6. The floor cleaning machine according to claim 1, **characterized in that** it comprises an electrostatic spraying unit for applying the electrolyzed water.

7. The floor cleaning machine according to claim 6, **characterized in that** the electrostatic spraying unit consists of corrosion-free parts and comprises electronic control and monitoring units, and has a spray lance or a spray bar with adjustable, electrostatic, air-assisted spray nozzles.

8. The floor cleaning machine according to any one of the claims 1 to 6, **characterized in that** the diamond electrodes are suitable of having an overpotential of at least 4 Volt.

9. The floor cleaning machine according to any one of the claims 1 to 8, **characterized in that** the water in the storage tank is mixed with 0.5 g to 8 g of common salt per liter of water.

## Revendications

1. Machine de nettoyage du sol pour le nettoyage non chimique et sans résidus, l'assainissement, la désinfection et la neutralisation d'odeurs des surfaces de sol,
**caractérisée en ce que**
elle comporte un générateur d'électrolyse avec au moins une cellule électrolytique dotée de bore, dans laquelle le générateur d'électrolyse produit de l'eau électrolysée, froide ou chaude, avec des radicaux oxydants, à partir de l'eau.

2. Machine de nettoyage du sol selon la revendication 1, **caractérisée en ce que** la cellule électrolytique est conçue avec une chambre ou avec deux chambres avec diaphragme.

3. Machine de nettoyage du sol selon l'une des revendications 1 ou 2, **caractérisée en ce qu'**elle comporte une pompe, un filtre, un débitmètre, un régulateur de pression, un capteur de débit d'eau électrique, une unité de commande électronique avec polarisation inverse automatique temporisée des électrodes, un dispositif de mesure redox, un réservoir de stockage d'eau avec un robinet d'arrivée et de sortie, des conduites d'eau, une clapet de non-retour et retour, une unité de commande programmable électronique avec des interrupteurs, un dispositif de contrôle électronique du niveau d'eau avec une soupape d'arrivée électronique, des leviers de mise en marche et d'arrêt, une horloge de chronométrage et de commutation, des conduites d'arrivée et d'évacuation d'eau vers des dispositifs de nettoyage à haute pression, un câble de mise à la terre et des lignes électriques avec des prises, un dispositif de mesure du temps de fonctionnement, un interrupteur d'arrêt d'urgence et un boîtier à fusibles.

4. Machine de nettoyage du sol selon l'une des revendications 1 à 2, **caractérisée en ce qu'**elle comporte un châssis de montage, de préférence mobile.

5. Machine de nettoyage du sol selon l'une des revendications 1 à 2, **caractérisée en ce qu'**elle comporte un réservoir d'eau avec une pompe de circulation pour le stockage intermédiaire.

6. Machine de nettoyage du sol selon la revendication 1, **caractérisée en ce qu'**elle comporte un système de pulvérisation électrostatique pour l'application de l'eau électrolysée.

7. Machine de nettoyage du sol selon la revendication 6, **caractérisée en ce que** le dispositif de pulvérisation électrostatique est constitué de pièces résistantes à la corrosion.

8. Machine de nettoyage du sol selon l'une des revendications 1 à 6, **caractérisée en ce que** les électrodes de diamant sont adaptées pour présenter une surtension d'au moins 4 volts.

9. Machine de nettoyage du sol selon l'une des revendications 1 à 8, **caractérisée en ce que** l'eau dans le réservoir de stockage est salée avec 0,5 g à 8 g de sel de cuisine par litre d'eau.
